# FASCICULE DE BREVET EUROPEEN

(11) **EP 1 940 772 B1**
(45) Date de publication et mention de la délivrance du brevet: **05.08.2009**
(21) Numéro de dépôt: 06831310.5
(22) Date de dépôt: 27.10.2006
(51) Int. Cl.: C07C 209/46, C07C 211/63, C07C 59/88, A61K 31/205

(54) **NOUVEAU PROCEDE DE PREPARATION DE SELS D'ACIDES ET D'AMMONIUM QUATERNAIRES**
NEUARTIGES VERFAHREN ZUR HERSTELLUNG VON QUATERNÄRER SÄURE UND AMMONIUMSALZEN
NOVEL METHOD FOR PREPARING QUATERNARY ACID AND AMMONIUM SALTS

(30) Priorité: 28.10.2005 FR 0511103
(43) Date de publication de la demande: 09.07.2008
(73) Titulaire: Fournier Laboratories Ireland Limited, Carrigtwohill, Co. Cork (IE)
(72) Inventeur: DELLIS, Philippe Résidence les Tourelles Bât. A, 06360 Eze (FR); NASAR, Kamel, 38780 Estrablin (FR)
(74) Mandataire: Hubert, Philippe
(86) Numéro de dépôt international: PCT/FR2006/051118
(87) Numéro de publication internationale: WO 2007/048986

(56) Documents cités:
- EP-A- 0 245 156
- WO-A-02/090307
- US-A1- 2005 148 594

## Description

L'invention concerne un nouveau procédé de synthèse d'un sel d'acide organique et d'une base, notamment un sel d'un acide de la famille des fibrates avec une base de type ammonium quaternaire, et plus particulièrement le sel de l'acide fénofibrique avec la choline.

### Art antérieur

Le fénofibrate 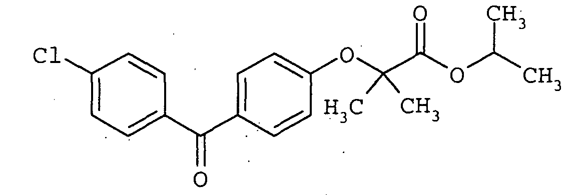 est un principe actif connu pour traiter les hypertriglycéridémies et les hypercholestérolémies. Ce composé est un ester isopropylique, mais en milieu biologique, l'ester est rapidement hydrolysé et conduit à l'acide fénofibrique qui est le métabolite actif du fénofibrate.

Il a été proposé récemment (US 2005/0148594) d'utiliser pour le traitement de ces maladies, une formulation galénique contenant l'acide fénofibrique lui-même en tant que principe actif et, plus préférentiellement, d'utiliser un sel de l'acide fénofibrique avec une base organique, notamment la choline. La préparation du sel de choline est décrite aux exemples 17A et 17B du document précité, et consiste à utiliser l'acide fénofibrique comme composé de départ, lequel est salifié par l'hydroxyde de choline, mis en oeuvre sous forme d'une solution dans le méthanol.

Le procédé actuellement le plus économique pour obtenir l'acide fénofibrique consiste à hydrolyser le fénofibrate qui est un produit commercial, qui peut être fabriqué par exemple par réaction de la 4-chloro-4'-hydroxybenzophénone avec le 2-bromo-2-méthylpropanoate d'isopropyle (EP0245156B1).

Des préparations de sels de choline sont aussi décrites dans la littérature. Par exemple, le brevet US3897485 décrit la préparation d'un sel avec un acide di-alkyl acétique, par combinaison de la choline base avec l'acide. De même le document WO96/16016 décrit la préparation d'un sel de ketoprofène et de choline et le document EP521393 décrit la préparation d'un sel de diclofénac et de choline, les procédés préconisés dans ces documents utilisant toujours l'acide comme produit de départ.

La présente invention a pour objet un nouveau procédé économique d'obtention d'un sel d'un acide fibrique et d'ammonium quaternaire de formule (IV) telle que définie ci-après, susceptible de conduire en une seule opération à un produit de grande pureté compatible avec une utilisation en thérapeutique humaine.

Ce procédé consiste essentiellement à faire réagir entre eux un ester α-halogéné de l'acide 2-méthylpropanoïque (II) et un phénol substitué (I) puis, sans isoler le produit intermédiaire ainsi formé, une base de type ammonium quaternaire (III), et peut être représenté par le schéma réactionnel suivant : 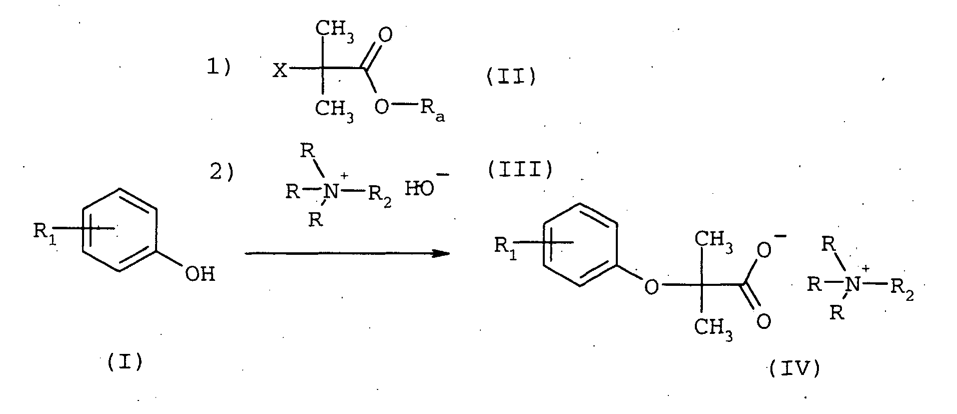 dans lequel :
R₁ représente un atome de chlore, un groupe 2-(4-chlorobenzoyl-amino)éthyl, un groupe 4-chlorobenzoyl ou un groupe 2,2-dichlorocyclopropyl et se trouve préférentiellement en position para du groupe OH ,
X représente un halogène, préférentiellement un atome de brome,
Rₐ représente un groupe alkyle en C₁-C₆, linéaire ou ramifié,
R₂ représente un groupe alkyle en C₁-C₄, linéaire ou ramifié ou un groupe hydroxyalkyle en C₁-C₄, linéaire ou ramifié,
R représente un groupe alkyle en C₁-C₃, linéaire.

Ainsi, l'originalité du procédé conforme à la présente invention consiste à faire réagir, en une seule opération, c'est-à-dire sans isoler le produit intermédiaire formé, les composés précurseurs de l'acide fibrique et un hydroxyde d'ammonium quaternaire. De façon tout à fait inattendue, on obtient ainsi directement un sel d'acide fibrique et d'ammonium quaternaire d'une pureté très élevée supérieure à 99,5 % susceptible d'être utilisé sans purification ultérieure comme principe actif d'un médicament à usage humain.

Ce procédé est particulièrement intéressant dans le cadre de la préparation d'un sel d'acide fénofibrique et d'ammonium quaternaire, en particulier d'un sel d'acide fénofibrique et de choline.

En effet, dans ce cas, contrairement aux procédés de préparation actuellement connus, il n'est pas nécessaire d'utiliser comme produit de départ, un acide fénofibrique provenant de l'hydrolyse du fénofibrate. Le procédé en résultant est par conséquent plus simple de mise en oeuvre et plus économique.

D'une façon générale, le procédé conforme à la présente invention permet en particulier de préparer un sel d'ammonium quaternaire d'un acide fibrique de formule : 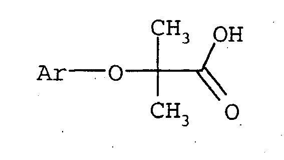 dans laquelle Ar représente un groupe phényle substitué en position para par un atome de chlore (il s'agit alors de l'acide clofibrique), par un groupe 4-chlorobenzoyle (il s'agit alors de l'acide fénofibrique), par un groupe 2-(4-chlorobenzoylamino)éthyle (il s'agit alors du bézafibrate) ou encore par un groupe 2,2-dichlorocyclopropyl (il s'agit alors du ciprofibrate).

Dans le cadre de la présente demande, par groupe alkyle en C₁-C₆, linéaire ou ramifié, on entend une chaîne hydrocarbonée saturée contenant 1 à 6 atomes de carbone, linéaire ou ramifiée, par exemple choisie parmi les groupes méthyle, éthyle, propyle, butyle, 1-méthyl-éthyle, 1-méthyl-propyle, 2-méthyl-propyle, 1,1-diméthyl-éthyle, pentyle ou encore hexyle.

Par groupe hydroxyalkyl en C₁-C₄ on entend une chaîne alkyle contenant 1 à 4 atomes de carbone telle que définie ci-dessus portant au moins un groupe OH directement lié à l'un quelconque des atomes de carbones.

D'une façon générale, les réactions chimiques intervenant dans la mise en oeuvre du procédé conforme à l'invention peuvent être conduites soit en présence d'un solvant, soit en absence de solvant, soit encore avantageusement en présence d'un solvant pour la deuxième réaction. En effet, l'étape finale du procédé doit être avantageusement conduite en présence d'un solvant et préférentiellement en présence d'un alcool pour obtenir un composé de grande pureté.

Selon un mode de réalisation préféré de l'invention, on utilisera un solvant unique pour la réaction finale et la purification du sel produit. Notamment, ce solvant est un propanol linéaire ou ramifié. Dans ces conditions la pureté du sel obtenu est supérieure à 99,5% lorsque l'on contrôle la pureté au moyen d'un dosage par HPLC (chromatographie liquide haute performance) pour la teneur en acide et par potentiométrie pour le dosage de l'ammonuim quaternaire.

Selon une caractéristique particulière, le procédé conforme à l'invention consiste :
- à faire réagir un phénol de formule (I) avec un ester α-halogéné de formule (II) avantageusement utilisé en excès par rapport aux conditions stoechiométriques, en présence d'une base, à une température comprise entre 80 et 160°C, pendant une durée de 1 à 8 heures ; puis
- à faire réagir dans le milieu réactionnel en résultant, en présence d'un solvant, de préférence après élimination des composés minéraux insolubles, un hydroxyde d'ammonium quaternaire de formule (III), à une température comprise entre 80 et 120°C, pendant une durée de 1 à 5 heures ; et
- à séparer le sel de formule (IV) ainsi formé.

De façon avantageuse, le solvant précité est un propanol linéaire ou ramifié (n-propanol ou isopropanol).

Le procédé conforme à la présente invention permet en particulier de préparer un sel d'acide fénofibrique et d'ammonium quaternaire par la mise en oeuvre des composés de formule (I) dans laquelle R₁ représente le groupe 4-chlorobenzoyl en position 4 du groupe hydroxy, de formule (II) dans laquelle Rₐ représente un groupe alkyle en C₁-C₃ linéaire ou ramifié et de formule (III) dans laquelle R représente un groupe méthyle et R₂ représente le groupe 2-hydroxyéthyle.

Un mode de réalisation préféré de l'invention consiste à faire réagir la 4-chloro-4'-hydroxybenzophénone avec un ester éthylique ou (iso)propylique de l'acide 2-bromo-2-méthyl-propanoïque et ajouter ensuite l'hydroxyde de choline pour obtenir directement, c'est-à-dire sans isolation du produit intermédiaire formé, dans une opération « one pot », le sel de choline de l'acide fénofibrique.

Selon un mode opératoire général du procédé selon l'invention, on prépare tout d'abord un mélange du phénol de formule 1, tel que défini précédemment, avec une quantité stoechiométriquement au moins équivalente de l'ester de formule II dans laquelle X représente un halogène, préférentiellement un atome de brome et Rₐ représente un groupe alkyle en C₁-C₆, linéaire ou ramifié. L'ester de formule II étant liquide à température ambiante il n'est généralement pas nécessaire d'utiliser un solvant à ce stade du procédé. Toutefois, si le réacteur utilisé ne permet pas d'obtenir une agitation suffisante, il est tout à fait possible de conduire la réaction en présence d'un solvant tel qu'un alcool ou une cétone, sans que ces deux exemples ne soient limitatifs. On utilisera cependant préférentiellement dans ce cas un solvant dont le point d'ébullition est au moins 80°C sous la pression atmosphérique.

Le mélange des réactifs de départ est ensuite porté à une température comprise entre 80 et 160°C, et on ajoute progressivement une base minérale de préférence en quantité sensiblement équimolaire relativement au composé de formule I. Cette base est par exemple un carbonate ou un bicarbonate de sodium ou de potassium ou encore un carbonate de calcium. On peut ajouter cette base sous forme de poudre ou de grains, lesdits produits étant généralement solides, mais on peut aussi ajouter cette base sous forme d'une solution très concentrée ou d'une suspension dans l'eau. La vitesse d'addition est classiquement assez rapide et n'est limitée que par le dégagement du gaz carbonique qui est produit par la réaction.

Le temps de réaction est compris entre 1 heure et 8 heures, au cours desquelles il est préférable d'éliminer par distillation l'eau introduite ou formée par la réaction.

Selon une caractéristique particulière, on ajoute ensuite un solvant des composés organiques contenus dans le milieu et on effectue avantageusement une filtration à chaud de façon à éliminer les composés minéraux insolubles. Ce solvant est par exemple un alcool et préférentiellement un propanol linéaire ou ramifié.

La solution maintenue en température est ensuite mise en présence du composé de formule III dans laquelle R₂ représente un groupe alkyle en C₁-C₄, linéaire ou ramifié ou un groupe hydroxyalkyle en C₁-C₄, linéaire ou ramifié, et R représente un groupe alkyle en C₁-C₃, linéaire. Ce composé peut être introduit pur ou en solution dans un solvant approprié tel que de préférence l'eau ou un alcool:

Le mélange réactionnel est agité à une température comprise entre 80 et 120°C, pendant 1 à 5 heures.

Si le composé de formule III a été introduit sous forme de solution dans l'eau, celle-ci est alors éliminée par distillation ou entraînement azéotropique.

Le mélange final sous forme d'une solution est alors filtré à chaud puis refroidi dans des conditions permettant la cristallisation du sel attendu qui est ensuite isolé par filtration sur filtre ou essoreuse et séché.

En suivant ce procédé selon l'invention, on obtient généralement le sel attendu avec une pureté conforme à une utilisation directe en thérapeutique, c'est-à-dire une pureté d'au moins 99,5 %.

L'invention concerne également le sel de pureté supérieure à 99,5 % obtenu selon le procédé de l'invention, ainsi que son utilisation en thérapeutique pour la fabrication de médicaments pour la médecine humaine. Ces médicaments sont de préférence formulés pour une absorption par voie orale, par exemple sous forme de gélules ou de comprimés. Ces préparations galéniques sèches, sous formes de tablettes, de comprimés pelliculés ou non pelliculés, ou de gélules, sont obtenues selon des méthodes connues de l'homme de métier, par exemple par mélange du sel avec des excipients de façon à obtenir par exemple un granulé qui peut être comprimé, ou introduit dans des gélules. Selon un mode préféré de réalisation de l'invention, le sel pur est un sel entre l'acide fénofibrique et un ammonium quaternaire, présent à raison d'une quantité comprise entre 40 et 180 mg par unité posologique. De préférence, l'ammonium quaternaire qui forme le sel avec l'acide fénofibrique est la choline.

La présente invention sera mieux comprise à la lecture de la description des modes opératoires suivants, présentés ici pour illustrer l'invention et qui ne doivent pas être considérés comme limitatifs.

### EXEMPLE I

### Acide 2-[4-(4-chlorobenzoyl)phénoxy]-2-méthyl-propanoïque, sel de choline

Un mélange de 1108 g (5,28 moles) de 2-bromo-2-méthylpropanoate d'isopropyle et 650 g (2,79 moles) de (4-chlorophényl)(4-hydroxyphényl)-méthanone est chauffé à 145°C sous bonne agitation, sous atmosphère d'azote, dans un réacteur de 5 l équipé pour travail à reflux ou distillation. On ajoute alors 448 g (3,24 moles) de carbonate de potassium et on porte la température du milieu réactionnel à 155°C. Le mélange réactionnel est agité à cette température pendant 4 heures. Pendant cette période, on recueille la phase aqueuse produite dans le distillat. La température du milieu de réaction est ramenée à 145°C et la pression interne du réacteur est abaissée progressivement de façon à éliminer par distillation l'excédent de réactif bromé. Ces conditions sont maintenues pendant environ 2 heures, au cours desquelles on recueille dans une recette tous les distillats. La température du mélange est ensuite abaissée à 120°C et on ajoute, le réacteur étant à pression atmosphérique, 1,95 l de propanol. La température du mélange est alors d'environ 80-90 °C et le mélange est filtré sous pression d'azote. Le solide résiduel est rincé sur le filtre par environ 0,75 l de propanol chaud. Les filtrats, maintenus en température, sont rassemblés dans le réacteur de 5 l et on ajoute progressivement 790 g (2,93 moles) d'une solution de choline hydroxyde à 45 % dans l'eau, puis 0,80 l de propanol. On porte ensuite le mélange réactionnel à ébullition à pression atmosphérique et on recueille le distillat produit jusqu'à obtenir environ 1,60 l d'un mélange propanol/eau/isopropanol. Le mélange est filtré sur un filtre clarificateur et le filtrat est refroidi progressivement sous agitation de façon à provoquer la cristallisation du sel, jusqu'à une température de 10°C environ. Le sel cristallisé est séparé et lavé sur essoreuse par 0,65 l de propanol froid, puis séché en étuve sous pression réduite.

On obtient ainsi 824 g du sel attendu sous forme de cristaux blancs (rendement = 70 %).

La pureté du sel obtenu est contrôlée par chromatographie HPLC en effectuant le dosage selon la méthode décrite pour le fénofibrate dans la pharmacopée européenne ou dans la pharmacopée US. La chromatographie effectuée par rapport à un échantillon de référence permet le dosage de l'acide fénofibrique. Le dosage de la choline présente dans le sel est effectué par potentiométrie.

L'analyse du composé montre une pureté supérieure à 99,5 % et l'absence d'impuretés dont le taux serait supérieur à 0,1 %. F = 213°C.

### EXEMPLE II

### Acide 2-[4-(4-chlorobenzoyl)phénoxy]-2-méthyl-propanoïque, sel de choline

Dans un réacteur de 1 l maintenu sous atmosphère d'azote, on prépare un mélange de 100 g (0,43 mole) de (4-chlorophényl)(4-hydroxy-phényl)méthanone et 148 g (0,82 mole) de 2-bromo-2-méthylpropanoate de méthyle. Le mélange est porté à 145°C sous bonne agitation et on ajoute 69 g (0,5 mole) de carbonate de potassium. Le milieu réactionnel est maintenu à 145°C sous bonne agitation pendant 3 heures, au cours desquelles on recueille en distillat l'eau formée par la réaction. Le réacteur est ensuite progressivement mis sous pression réduite de façon à éliminer par distillation l'excédent de réactif bromé. Le mélange est ensuite refroidi vers 100°C et on ajoute 300 ml de n-propanol. Le mélange obtenu est agité 10 mn à 90°C puis filtré à cette température de façon à éliminer les sels minéraux insolubles. Le solide résiduel est rincé par 100 ml de n-propanol chaud, que l'on joint aux filtrats précédents. La solution obtenue est placée dans le réacteur de 1l sous atmosphère d'azote et on ajoute 121,5 g (0,45 mole) d'une solution de choline hydroxyde à 45 % dans l'eau. Le mélange réactionnel est agité pendant 3 heures à léger reflux du solvant puis on distille environ 240 ml de solvant tout en ajoutant dans le réacteur 130 ml de n-propanol. Le contenu du réacteur est ensuite filtré sur filtre clarificateur puis refroidi lentement jusqu'à environ 15°C. La suspension obtenue est filtrée sur essoreuse et le solide isolé est rincé par 100 ml de n-propanol froid, puis séché en étuve sous vide.

On obtient ainsi 122 g du sel attendu sous forme d'un solide cristallisé blanc. (Rendement = 67,5 %).

La pureté du sel obtenu est supérieure à 99,5 %.

### EXEMPLE III

### Acide 2-[4-(4-chlorobenzoyl)phénoxyl-2-méthyl-propanoïque, sel de choline

La réaction est effectuée de façon analogue à celle décrite pour l'exemple II mais en utilisant 159 g de 2-bromo-2-méthylpropanoate d'éthyle.

On obtient 127 g du sel attendu, sous forme d'une poudre cristalline blanche soit un rendement de 70 %. Le sel présente une pureté supérieure à 99,8 %.

## Revendications

1. Procédé de préparation d'un sel d'acide fibrique et d'ammonium quaternaire de formule IV : 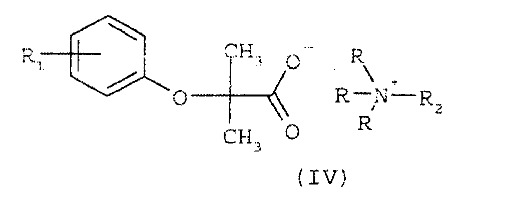 dans laquelle :
R₁ représente un atome de chlore, un groupe 2-(4-chlorobenzoyl-amino)éthyl, un groupe 4-chlorobenzoyl ou un groupe 2,2-dichlorocyclopropyl,
R₂ représente un groupe alkyle en C₁-C₄, linéaire ou ramifié ou un groupe hydroxyalkyle en C₁-C₄, linéaire ou ramifié,
R représente un groupe alkyle en C₁-C₃, linéaire,
**caractérisé en ce qu'**il peut être représenté par le schéma réactionnel suivant : 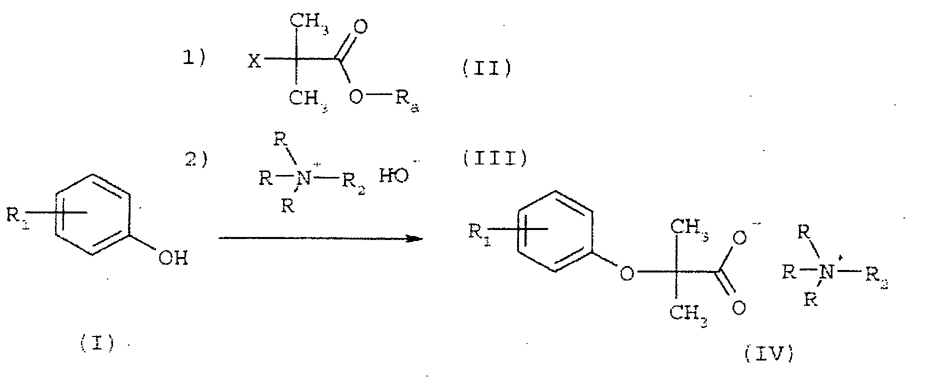 et **en ce qu'**il est mis en oeuvre en une seule opération au départ d'un phénol de formule (I) dans laquelle R₁ est tel que défini précédemment, d'un ester α-halogéné de formule (II) dans laquelle X représente un halogène et Rₐ représente un groupe alkyle en C₁-C₆, linéaire ou ramifié, et d'un hydroxyde d'ammonium quaternaire de formule (III), dans laquelle R et R₂ sont tels que définis précédemment.

2. Procédé selon la revendication 1, **caractérisé en ce que** dans le composé de formule (I) précitée R₁ se trouve en position para de l'oxygène.

3. Procédé se!on la revendication 1 ou 2, **caractérisé en ce que** dans le composé de formule (I) précitée, R₁ représente un groupe 4-chlorobenzoyl, situé en position para de l'oxygène.

4. Procédé selon l'une quelconque des revendications 1 à 3, **caractérisé en ce que** dans le composé de formule (III) précitée, R représente un groupe méthyle et R₂ représente un groupe 2-hydroxyéthyle.

5. Procédé selon l'une quelconque des revendications 1 à 4, **caractérisé en ce que** dans le composé de formule (II) précitée, X est un atome de brome.

6. Procédé selon l'une quelconque des revendications 1 à 5, **caractérisé en ce qu'**il consiste :
- à faire réagir un phénol de formule (I) avec un ester α-halogéné de formule (II) avantageusement utilisé en excès par rapport aux conditions stoechiométriques, en présence d'une base, à une température comprise entre 80 et 160°C, pendant une durée de 1 à 8 heures ; puis
- à faire réagir dans le milieu réactionnel en résultant, en présence d'un solvant, un hydroxyde d'ammonium quaternaire de formule (III), à une température comprise entre 80 et 120°C, pendant une durée de 1 à 5 heures ; et
- à séparer le sel de formule (IV) ainsi formé.

7. Procédé selon l'une quelconque des revendications 1 à 5, **caractérisé en ce qu'**il consiste :
- à faire réagir un phénol de formule (I) avec un ester α-halogéné de formule (II) avantageusement utilisé en excès par rapport aux conditions stoechiométriques, en présence d'une base, à une température comprise entre 80 et 160°C, pendant une durée de 1 à 8 heures ; puis
- à faire réagir dans le milieu réactionnel en résultant, en présence d'un solvant, après élimination des composés minéraux insolubles, un hydroxyde d'ammonium quaternaire de formule (III), à une température comprise entre 80 et 120°C, pendant une durée de 1 à 5 heures ; et
- à séparer le sel de formule (IV) ainsi formé.

8. Procédé selon la revendication 6 ou 7, **caractérisé en ce qu'**il consiste :
- à faire réagir un phénol de formule (I) avec une quantité stoéchiométriquement au moins équivalente de l'ester de formule (II), à une température comprise entre 80 et 160°C, en présence d'une base minérale, pendant une durée de 1 à 8 heures,
- à ajouter au milieu réactionnel un solvant, tel que par exemple un alcool,
- à effectuer une filtration à chaud de façon à éliminer les composés minéraux insolubles,
- à faire réagir la solution obtenue avec un hydroxyde d'ammonium quaternaire de formule (III), à une température comprise entre 80 et 120°C, pendant 1 à 5 heures ;
- à filtrer à chaud puis refroidir dans des conditions permettant la cristallisation du sel attendu qui est ensuite isolé par filtration et séché, le sel ainsi obtenu présentant une pureté d'au moins 99,5 %.

9. Procédé selon l'une des revendications 6 à 8, **caractérisé en ce que** le solvant précité est un propanol linéaire ou ramifié.

10. Procédé selon l'une des revendications 6 à 9, **caractérisé en ce que** la base minérale précitée est choisie parmi un carbonate ou un bicarbonate de sodium ou de potassium et un carbonate de calcium.

## Claims

1. Process for the preparation of a quaternary ammonium salt of a fibric acid of formula IV: 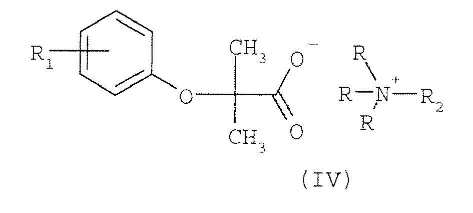 in which:
R₁ is a chlorine atom, a 2-(4-chlorobenzoylamino)ethyl group, a 4-chlorobenzoyl group or a 2,2-dichlorocyclopropyl group,
R₂ is a linear or branched C₁-C₄ alkyl group or a linear or branched C₁-C₄ hydroxyalkyl group,
R is a linear C₁-C₃ alkyl group,
**characterized in that** it can be represented by the following reaction scheme: 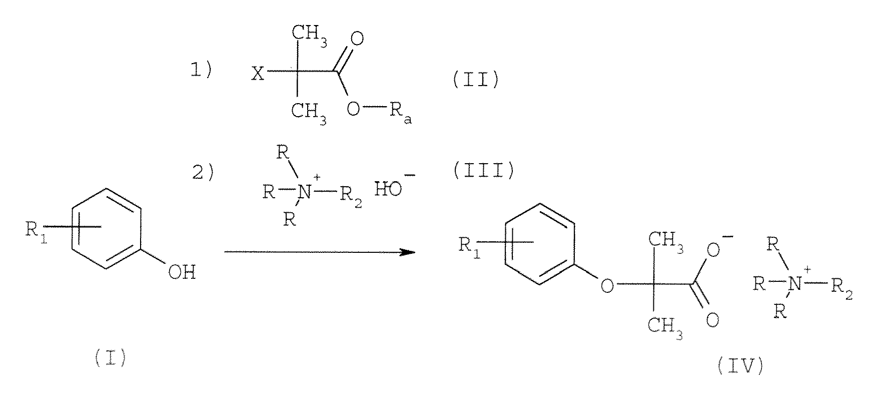 and **in that** it is carried out in a single operation starting from a phenol of formula (I), in which R₁ is as defined above, an ester of formula (II), in which X is a halogen and Rₐ is a linear or branched C₁-C₆ alkyl group, and a quaternary ammonium hydroxide of formula (III), in which R and R₂ are as defined above.

2. Process according to claim 1, **characterized in that**, in the compound of formula (I) given above, R₁ is located in the para position relative to the oxygen.

3. Process according to claim 1 or 2, **characterized in that**, in the compound of formula (I) given above, R₁ is a 4-chlorobenzoyl group located in the para position relative to the oxygen.

4. Process according to any one of claims 1 to 3, **characterized in that**, in the compound of formula (III) given above, R is a methyl group and R₂ is a 2-hydroxyethyl group.

5. Process according to any one of claims 1 to 4, **characterized in that**, in the compound of formula (II) given above, X is a bromine atom.

6. Process according to any one of claims I to 5, **characterized in that** it consists in:
- reacting a phenol of formula (I) with an α-halogenated ester of formula (II), advantageously used in excess relative to stoichiometric conditions, in the presence of a base, at a temperature between 80 and 160°C, for a period of 1 to 8 hours; then
- reacting a quaternary ammonium hydroxide of formula (III) with the resulting reaction medium, in the presence of a solvent, at a temperature between 80 and 120°C, for a period of 1 to 5 hours; and - separating off the salt of formula (IV) formed.

7. Process according to any one of claims 1 to 5, **characterized in that** it consists in:
- reacting a phenol of formula (I) with an α-halogenated ester of formula (II), advantageously used in excess relative to stoichiometric conditions, in the presence of a base, at a temperature between 80 and 160°C, for a period of 1 to 8 hours; then
- reacting a quaternary ammonium hydroxide of formula (III) with the resulting reaction medium, in the presence of a solvent, preferably after removal of the insoluble mineral compounds, at a temperature between 80 and 120°C, for a period of 1 to 5 hours; and
- separating off the salt of formula (IV) formed.

8. Process according to claim 6 or 7, **characterized in that** it consists in:
- reacting a phenol of formula (I) with a stoichiometrically at least equivalent amount of the ester of formula (II) at a temperature between 80 and 160°C, in the presence of a mineral base, for a period of I to 8 hours,
- adding a solvent, such as for example an alcohol, to the reaction medium.
- carrying out a hot filtration to remote the insoluble mineral compounds.
- reacting the resulting solution with a quaternary ammonium hydroxide of formula (III) at a temperature between 80 and 120°C for 1 to 5 hours, and
- filtering the mixture hot and subsequently cooling it under conditions that allow the expected salt to crystallize, after which the crystals are filtered off and dried to give the expected salt with a purity of at least 99.5%.

9. Process according to one of claims 6 to 8, **characterized in that** the aforementioned solvent is a linear or branched propanol.

10. Process according to one of claims 6 to 9, **characterized in that** the aforementioned mineral base is selected from sodium or potassium carbonate or bicarbonate and calcium carbonate.

## Patentansprüche

1. Verfahren zur Herstellung eines Salzes aus Fibrinsäure und quaternärem Ammonium der Formel IV: 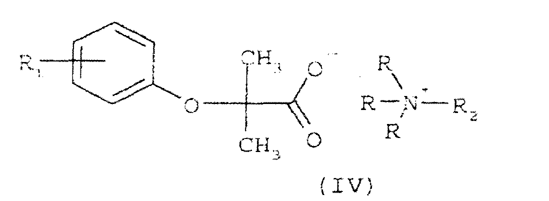 worin:
R₁ ein Chloratom, eine 2-(4-Chlorobenzoyl-amino)ethyl-Gruppe, eine 4-Chlorobenzoyl-Gruppe oder eine 2,2-Dichlorocyclopropyl-Gruppe darstellt,
R₂ eine lineare oder verzweigte C₁-C₄-Alkylgruppe oder eine lineare oder verzweigte C₁-C₄-Hydroxyalkylgruppe darstellt,
R eine lineare C₁-C₃-Alkylgruppe darstellt,
**dadurch gekennzeichnet, daß** es durch folgendes Reaktionsschema dargestellt werden kann: 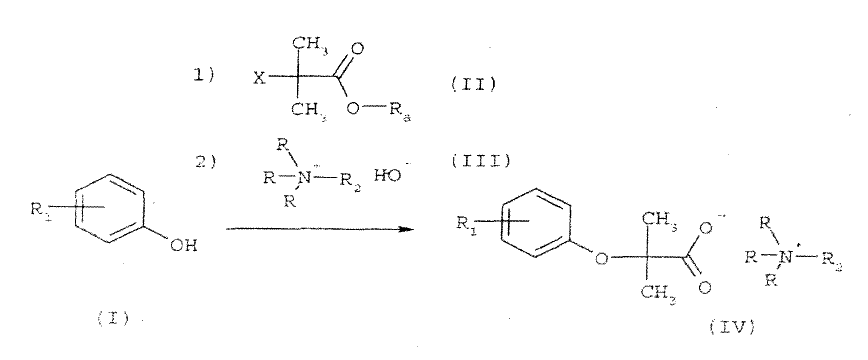 und daß es in einem einzigen Schritt anhand eines Phenols der Formel (I), worin R₁ derart wie oben definiert ist, eines α-halogenierten Esters der Formel (II), worin X ein Halogen und Rₐ eine lineare oder verzweigte C₁-C₆-Alkylgruppe darstellt, sowie eines quaternären Ammoniumhydroxids der Formel (III), worin R und R₂ derart wie oben definiert sind, durchgeführt wird.

2. Verfahren nach Anspruch 1, **dadurch gekennzeichnet, daß** sich bei der Verbindung der vorgenannten Formel (I) R₁ in para-Stellung des Sauerstoffs befindet.

3. Verfahren nach Anspruch 1 oder 2, **dadurch gekennzeichnet, daß** bei der Verbindung der vorgenannten Formel (I) R₁ eine 4-Chlorobenzoyl-Gruppe in para-Stellung des Sauerstoffs darstellt.

4. Verfahren nach einem der Ansprüche 1 bis 3, **dadurch gekennzeichnet, daß** bei der Verbindung der vorgenannten Formel (III) R eine Methylgruppe und R₂ eine 2-Hydroxyethylgruppe darstellt.

5. Verfahren nach einem der Ansprüche 1 bis 4, **dadurch gekennzeichnet, daß** bei der Verbindung der vorgenannten Formel (II) X ein Bromatom ist.

6. Verfahren nach einem der Ansprüche 1 bis 5, **dadurch gekennzeichnet, daß** es darin besteht:
- ein Phenol der Formel (I) mit einem α-halogenierten Ester der Formel (II), das bezogen auf die stöchiometrischen Bedingungen vorteilhafterweise im Überschuß verwendet wird, in Anwesenheit einer Base, bei einer Temperatur zwischen 80 und 160°C, für einen Zeitraum von 1 bis 8 Stunden reagieren zu lassen, anschließend
- in dem hieraus hervorgehenden Reaktionsmedium, in Anwesenheit eines Lösungsmittels, ein quaternäres Ammoniumhydroxid der Formel (III) bei einer Temperatur zwischen 80 und 120°C, für einen Zeitraum von 1 bis 5 Stunden reagieren zu lassen, und
- das so gebildete Salz der Formel (IV) abzuscheiden.

7. Verfahren nach einem der Ansprüche 1 bis 5, **dadurch gekennzeichnet, daß** es darin besteht:
- ein Phenol der Formel (I) mit einem α-halogenierten Ester der Formel (11), das bezogen auf die stöchiometrischen Bedingungen vorteilhafterweise im Überschuß verwendet wird, in Anwesenheit einer Base, bei einer Temperatur zwischen 80 und 160°C, für einen Zeitraum von 1 bis 8 Stunden reagieren zu lassen, anschließend
- in dem hieraus hervorgehenden Reaktionsmedium, in Anwesenheit eines Lösungsmittels, nach Entfernen der unlöslichen mineralischen Verbindungen, ein quaternäres Ammoniumhydroxid der Formel (III) bei einer Temperatur zwischen 80 und 120°C für einen Zeitraum von 1 bis 5 Stunden reagieren zu lassen, und
- das so gebildete Salz der Formel (IV) abzuscheiden.

8. Verfahren nach Anspruch 6 oder 7, **dadurch gekennzeichnet, daß** es darin besteht:
- ein Phenol der Formel (I) mit einer stöchiometrisch wenigstens gleichwertigen Menge des Esters der Formel (II) bei einer Temperatur zwischen 80 und 160°C, in Anwesenheit einer mineralischen Base, für einen Zeitraum von 1 bis 8 Stunden reagieren zu lassen,
- dem Reaktionsmedium ein Lösungsmittel, wie zum Beispiel einen Alkohol zuzugeben,
- eine Heißfiltration durchzuführen, so daß die unlöslichen mineralischen Verbindungen entfernt werden,
- die erhaltene Lösung mit einem quaternären Ammoniumhydroxid der Formel (III) bei einer Temperatur zwischen 80 und 120°C für 1 bis 5 Stunden reagieren zu lassen,
- heiß zu filtern, dann abzukühlen unter Bedingungen, die die Kristallisation des erwarteten Salzes ermöglichen, das anschließend durch Filtration abgetrennt und getrocknet wird, wobei das so erhaltene Salz eine Reinheit von wenigstens 99,5 % aufweist.

9. Verfahren nach einem der Ansprüche 6 bis 8, **dadurch gekennzeichnet, daß** das vorgenannte Lösungsmittel ein lineares oder verzweigtes Propanol ist.

10. Verfahren nach einem der Ansprüche 6 bis 9, **dadurch gekennzeichnet, daß** die vorgenannte mineralische Base aus einem Natrium- oder Kaliumcarbonat oder - bicarbonat oder einem Kalziumcarbonat ausgewählt ist.
